# EUROPEAN PATENT SPECIFICATION

(11) **EP 2 413 947 B1**
(45) Date of publication and mention of the grant of the patent: **29.04.2020**
(21) Application number: 10759288.3
(22) Date of filing: 30.03.2010
(51) Int. Cl.: A61K 38/00, A61K 38/08, A61K 9/70

(54) **METHODS AND COMPOSITIONS FOR TREATING SKIN CONDITIONS ASSOCIATED WITH VASCULAR HYPER-REACTIVITY**
VERFAHREN UND ZUSAMMENSETZUNG ZUR BEHANDLUNG VON MIT GEFÄSSHYPERREAKTIVITÄT ASSOZIIERTEN HAUTLEIDEN
PROCÉDÉ ET COMPOSITION DE TRAITEMENT DES AFFECTIONS DE LA PEAU ASSOCIÉES À UNE HYPERRÉACTIVITÉ VASCULAIRE

(30) Priority: 01.04.2009 US 165701 P
(43) Date of publication of application: 08.02.2012
(73) Proprietor: Revance Therapeutics, Inc., Newark, CA 94560 (US)
(72) Inventor: HO, Connie L., Palo Alto California 94301 (US); DU, Alice, San Jose California 94301 (US); BROWNE, Dan, Palo Alto California 94301 (US)
(74) Representative: J A Kemp LLP
(86) International application number: PCT/US2010/029183
(87) International publication number: WO 2010/114828

(56) References cited:
- WO-A2-2004/006954
- WO-A2-2006/094263
- WO-A2-2008/082889
- US-A1- 2005 010 161
- US-A1- 2005 214 325
- US-A1- 2005 214 325
- US-A1- 2007 178 121
- US-A1- 2008 112 981
- US-A1- 2008 112 981
- US-A1- 2008 299 220
- YURAITIS ET AL.: 'Botulinum toxin for the treatment of facial flushing.' DERMATOL. SURG. vol. 30, no. 1, 2004, pages 102 - 104, XP055094276

## Description

### FIELD OF THE INVENTION

The present invention relates to a topical composition for use in a method of treating the treatment of skin conditions characterized by vascular hyper-reactivity, or hyper-reactive vascular dilation. In particular, the present invention relates to a topical formulation of botulinum neurotoxin for treating conditions such as rosacea, or for preventing or slowing progression of rosacea.

### BACKGROUND

Rosacea is a chronic condition characterized by facial redness that can progress into dilated superficial blood vessels in the face, red-domed papules, burning and itching sensations, and a red, bulbous nose. Rosacea affects over 14 million Americans (National Rosacea Society, 2009), and the peak age of onset is between 30 and 60 years.

Rosacea may be classified into at least four subtypes with varying symptoms, and a patient may concurrently be afflicted with one or more subtypes (Wilkin et al. (2004), J Am Acad Dermatol, Vol. 50 (6): 907-12). Erythematotelangiectatic rosacea is characterized by a permanent erythema *(i.e.* redness) of the cheeks, forehead, and nose and the patient has a tendency to flush and blush easily. Typically, small blood vessels are visible near the surface of the skin (*e.g*. telangiectasias) and the individual may experience burning or itching sensations. Papulopustular rosacea can be easily confused with acne due to the presence of some permanent redness across the central face with red papules, some of which are pus filled (pustules). Phymatous rosacea is the subtype most commonly associated with rhinophyma, an enlargement of the nose due to sebaceous gland hyperplasia. Symptoms of phymatous rosacea can include thickening skin, irregular surface nodularities, and enlargement, which can also affect the chin, forehead, cheeks, eyelids, and ears (Jansen and Plewig G (1998), Facial Plast Surg, Vol. 14 (4): 241-53). A fourth subtype, known as ocular rosacea, affects primarily the eyes and is characterized by red, irritated eyes, swollen eyelids, recurrent styes, and sensations of itching and burning.

The symptoms of rosacea are socially disabling for many patients and the condition may last for years and rarely reverses itself without treatment. Treatments are typically individually tailored due to the varying type and severity of symptoms. Various oral and topical medications, including antibiotics, retinoids, benzoyl peroxide, and beta blockers, are initially prescribed to bring the condition under control, which may take up to one to two years. Long term treatment is often necessary to maintain remission of the condition. The more advanced stages of rosacea can require laser, intense light source, or surgical treatment to remove visible superficial blood vessels, reduce extensive redness, or ameliorate disfigurements of the nose.

The currently available treatments for rosacea and associated symptoms are not cost-effective and can carry a substantive medical risk. Further, while topical treatments have the advantage of avoiding unwanted systemic toxicity, the effectiveness of topical treatments can be limited by, for example, large areas of affected skin, the varying depths of the skin microvasculature, and individual variations in patient skin.

More effective treatments for skin conditions associated with vascular hyper- reactivity are needed, including treatments effective for rosacea as well as treatments effective for preventing the progression of rosacea. US 2008/112981 discloses methods of using clostridial toxins and other biological agents to thin skin and control fine wrinkles in humans.

### SUMMARY OF THE INVENTION

The present invention provides compositions for use in a method of treating, reducing, or ameliorating vasodilation in the cutaneous microvasculature. Thus, the present invention is useful for treating or ameliorating the vascular hyper-reactivity that characterizes conditions such as rosacea. Further, the compositions of the invention may alleviate symptoms such as chronic or episodic flushing and blushing, and prevent their progression to rosacea.

The present invention provides a topical composition for use in a method for treating a patient having a skin condition characterized by cutaneous vascular hyper-reactivity, such as chronic or episodic flushing or blushing, or rosacea. The method comprises applying the topical composition to the affected area(s) of the patient's skin. The topical composition comprises an effective amount of a botulinum neurotoxin for decreasing vasodilation in the cutaneous microvasculature, and a carrier for effectively transporting the botulinum toxin to the cutaneous microvasculature. For example, the botulinum neurotoxin is formulated with a positively-charged peptide carrier (e.g., polylysine) having one or more protein transduction domains (or transporter domains), such as HIV-TAT or reverse HIV-TAT amino acid sequences. The method may involve a single application of topical botulinum toxin to the affected regions, or in certain embodiments, may involve repeated application.

In particular, the invention provides a topical composition for use in a method of treating a patient having a skin condition characterized by cutaneous vascular hyper-reactivity, wherein the topical composition is applied to area(s) of the patient's skin affected with said vascular hyper-reactivity, and wherein the topical composition comprises an amount of a botulinum neurotoxin effective for decreasing vasodilation in the cutaneous microvasculature, wherein said botulinum neurotoxin is non-covalently associated with a positively charged peptide carrier selected from Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID NO:3); Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO:4), where n is an integer from 5 to 20; and Arg-Lys-Lys-Arg-Arg-Gln-Arg-Arg-Arg-Gly-(Lys)15-Gly-Arg-Lys-Lys-Arg-Arg-Gln-Arg-Arg-Arg (SEQ ID NO:5), for transporting the botulinum neurotoxin to the cutaneous microvasculature.

The invention allows for effective amounts of botulinum toxin to be controllably applied to affected regions of the skin, and controllably delivered to the underlying microvasculature. The invention thereby provides a safe, effective, and comfortable manner of treating vascular hyper-reactivity in skin, so as to treat, ameliorate, or prevent progression of rosacea, and/or to prevent chronic or episodic vascular hyper-reactivity from progressing to a condition such as rosacea.

The present disclosure provides compositions, formulations, kits, and/or other vehicles or devices for topically administering botulinum neurotoxin for the treatment of cutaneous vascular hyper-reactivity. For example, the disclosure provides creams, lotions, gels, and patches that allow the botulinum neurotoxin and carrier to be conveniently, safely, comfortably and/or controllably applied to the affected area(s) of the patient's skin.

The present invention also provides:
- a skin patch comprising the topical composition of the invention and a patch for the skin;
- a kit for treating a skin condition characterised by vascular hyper-reactivity, which kit comprises a topical composition of the invention, further comprising one or more components selected from a gentle skin cleanser(s) for removing the excess botulinum toxin composition following treatment, a redness reducing lotion, one or more additional topical treatments for rosacea, and sun protection products with an SPF of 15 or greater;
- use of a composition of the invention, in the manufacture of a medicament for treating a patient having a skin condition characterized by cutaneous vascular hyper-reactivity; and
- a non-therapeutic method for treating a patient having a skin condition characterized by cutaneous vascular hyper-reactivity, comprising applying a topical composition to the affected area(s) of the patient's skin, wherein the topical composition is the present invention.

### DETAILED DESCRIPTION OF THE INVENTION

The present invention provides compositions for use in a method of treating, reducing, or ameliorating vasodilation in the cutaneous microvasculature. Thus, the present invention is useful for treating or ameliorating the vascular hyper-reactivity that characterizes conditions such as rosacea. The method involves applying the topical composition to affected area(s) of the patient's skin. The topical composition comprises an effective amount of a botulinum neurtotoxin for decreasing vasodilation in cutaneous microvasculature, and a carrier for effectively transporting the botulinum toxin to the cutaneous microvasculature.

Botulinum toxin is known to mediate the effects of acetylcholine in soft tissue (generally via injection) and has been used effectively to decrease sweating and inhibit muscle spasm. However, in accordance with the present disclosure, botulinum toxin is administered topically to decrease vasodilation in the cutaneous microvasculature, to thereby reduce vascular hyper-reactivity in skin. The invention decreases or eliminates chronic or episodic flushing or blushing as solitary conditions, and/or, delays or prevents the progressive disease of rosacea, e.g., flushing, papules, and eventual rhinophyma.

Vasoconstriction may be mediated by inhibiting the release of vasoactive intestinal peptide (VIP), calcitonin gene-related-peptide (CGRP) or other peptides contained in presynaptic vesicles. Vasodilator neuropeptides are involved in vascular tone of the peripheral circulation by enhancing nitric oxide formation in the microvasculature of the human dermis. Without being bound by theory, botulinum neurotoxin may decrease vasodilation in cutaneous microvasculature by blocking the release of vasodilator neuropeptides, such as CGRP and VIP that are co-released with acetylcholine from sudomotor nerve terminals. Therefore, inhibiting the release of vasodilators with botulinum neurotoxin can lead to vasoconstriction or the avoidance of vasodilation and the avoidance of blushing and flushing of the skin, and thus the subsequent development of the progressive disease of rosacea.

Existing treatments for rosacea include topical and oral antibiotics, benzoyl peroxide, retinoids, beta blockers, cortisone cream, and laser or intense pulse light treatments. All of these treatments are associated with one or more disadvantages. Topical and oral antibiotics have the risk of promoting bacterial multidrug resistance and allergic reactions, which can result in death. Benzoyl peroxide and retinoids can be irritating to the skin, and cortisone creams are limited to short-duration uses and can cause a rebound flare up when discontinued. Beta blockers can produce unwanted side effects, such as fatigue and sexual dysfunction. Associated risks with laser and intense pulse light treatments include scarring, bruising, longer recovery, higher cost, and pain.

The topical botulinum neurotoxin compositions and associated treatments as described herein overcome disadvantages associated with the existing treatments for rosacea. The compositions can be applied over a large area, do not cause pain, provide a prolonged period of relief, and eliminate the risk of overuse of antibiotics in a chronic condition. In addition, the topical formulation overcomes some of the technical difficulties of using injectable botulinum neurotoxin. For instance, administration of injectable botulinum neurotoxin is operator dependent and can be injected too deeply into facial nerves and muscles resulting in a functional impairment and facial asymmetry. It is exceedingly challenging to inject botulinum neurotoxin in a large surface area of the face, neck and chest, which is required to adequately treat vascular hyper-reactive skin conditions. The injections are not contiguous and therefore rely on diffusion for even application. Injections of botulinum neurotoxin into the skin of patients with vascular hyper-reactivity are likely to make the skin more sensitive, red and bruised, thus irritating the particular skin condition to be treated. The compositions of the invention offer a dilute topical form of botulinum toxin that is designed to target the superficial layers of skin, and which can be applied evenly over large areas.

The invention thereby provides a safe, effective, comfortable, and/or convenient manner of treating vascular hyper-reactivity in skin, including chronic or episodic vascular hyper-reactivity, so as to treat or ameliorate rosacea, and/or to prevent the chronic or episodic vascular hyper-reactivity from progressing to a condition such as rosacea.

### Conditions Characterized by Cutaneous Vascular Hyper-Reactivity

The patient to be treated has a skin condition associated with cutaneous vascular hyper-reactivity, such as, without limitation, flushing, blushing, and rosacea.

Flushing is an episodic attack of redness of the skin together with a sensation of warmth or burning of the face, neck, and less frequently the upper trunk and abdomen. It is the transient nature of the attacks that distinguishes flushing from the persistent erythema of photosensitivity, acute contact reactions, or solar elastosis (Greaves (1998) Flushing and flushing syndromes, rosacea and perioral dermatitis. In: Champion RH, et al, eds. Rook/Wilkinson/Ebling textbook of dermatology, 6th ed., Vol. 3: 2099-2104).

Blushing is related to flushing but is typically less intense and limited to redness of the face or cheeks. Repeated flushing over a prolonged period of time can lead to telangiectasia and to rosacea of the face (Greaves (1998) Flushing and flushing syndromes, rosacea and perioral dermatitis. In: Champion RH, et al, eds. Rook/Wilkinson/Ebling textbook of dermatology, 6th ed., Vol. 3: 2099-2104). The initial blushing and flushing of rosacea may lead to advanced stages or subtypes of rosacea characterized by persistent redness, papules, cysts, and eventual rhinophyma.

The flushing or blushing may result from an underlying condition or disease including, but not limited to, social phobia (*e.g.* generalized anxiety disorder, panic disorder), alcoholism, menopause and premenstrual syndrome, diabetes, lung disorders or diseases (*e.g.* emphysema, chronic bronchitis), insect sting allergies, pregnancy, mastocytosis, Soto's Syndrome, Zollinger-Ellison syndrome, Cushing's syndrome, Hodgkin's disease, septicemia, yellow fever, niacin derivatives, acipimox, Acute Stress Disorder, adrenal cancer, anticholinergic syndrome, hemorrhagic fever, autonomic dysreflexia syndrome, carcinoid syndrome, hyperadrenalism, hyperemia, hyperglycemia, hypergonadotropic ovarian failure, gastro-enteropancreatic neuroendocrine tumor, hypermagnesaemia, hyperthyroidism, medullary thyroid carcinoma, mitral valve disease, opioid withdrawal, neuroblastoma, niacin toxicity, paraneoplastic autoimmune syndromes, pituitary gland diseases, spinal autonomic dysreflexia, thymus cancer, and thyroid cancer.

In certain embodiments, the patient has (e.g., has been diagnosed with) rosacea, such as one or more of erythematotelangiectatic rosacea, papulopustular rosacea, phymatous rosacea, and/or ocular rosacea. In these or other embodiments, the patient has one or more symptoms of rosacea. The one or more symptoms of rosacea include erythema, flushing, blushing, telangiectasias, papules, pustules, rhinophyma, burning sensations, and itching sensations. Such one or more symptoms are improved or ameliorated in accordance with the invention.

In certain embodiments, the patient has rosacea, but does not yet have rhinophyma, which frequently occurs with phymatous rosacea or in advanced stages of rosacea. Rhinophyma is a slow growing benign tumor on the nose caused by the hypertrophy of sebaceous glands at the tip of the nose. In accordance with these embodiments, the compositions of the present invention may prevent the development of rhinophyma.

### Botulinum Toxin

The compositions and non-therapeutic method of the invention involve botulinum neurotoxin, formulated for topical delivery as described herein. Botulinum toxins or botulinum neurotoxins are neurotoxins produced by the gram-positive bacteria *Clostridium botulinum* and are one of the most lethal substances known to man. The neurotoxins are well known to produce paralysis of muscles by preventing release of acetylcholine from pre-synaptic nerve terminals at the neuromuscular junction. The neurotoxins can inhibit synaptic transmission at cholinergic synapses of the autonomic nervous system as well.

Botulinum toxin is classified into eight neurotoxins that are serologically related, but distinct. Of these, seven can cause paralysis, namely botulinum neurotoxin serotypes A, B, C, D, E, F and G. Each of these serotypes is distinguished by neutralization with type-specific antibodies. Each serotype can be isolated from the bacteria or produced recombinantly. The molecular weight of the purified, active botulinum toxin protein molecule for all seven of the toxin serotypes is about 150 kDa, which includes a 100 kDa heavy chain and a 50 kDa light chain. As released by the bacterium, the botulinum toxins are complexes comprising the 150 kDa botulinum toxin protein molecule along with associated non-toxic proteins. The botulinum toxin type A complex can be produced by *Clostridia* as 900 kDa, 500 kDa and 300 kDa forms. Botulinum toxin types Band C can be produced as a 700 kDa or 500 kDa complex. Botulinum toxin type D is produced as both 300 kDa and 500 kDa complexes, while botulinum toxin types E and F are produced as only approximately. 300 kDa complexes. The nontoxic proteins associated with the 150 kDa botulinum toxin are thought to provide stability against denaturation and provide protection against digestive acids when toxin is ingested. While the complexed forms of the botulinum neurotoxins described above may be used in connection with the compositions and methods described herein, in certain embodiments of the invention, the botulinum toxin is purified (*e.g*. not associated with accessory proteins) and has a molecular weight of about 150kDa.

The botulinum toxin may be selected from botulinum toxin serotypes A, B, C, D, E, F, and G. In certain embodiments the botulinum toxin is botulinum toxin serotype A, including Types A1, A2 and/or Type A3. Exemplary Type A producing strains include the Hall Strain (Type A1), Kyoto F (Type A2), and NCTC 2916 (Type A1). See Jacobson et al., Analysis ofNeurotoxin Cluster Genes in Clostridium botulinum Strains Producing Botulinum Neurotoxin Serotype A Subtypes, Appl. Environ. Microbial. 74(9):2778-2786 (2008). Botulinum toxin suitable for use in the compositions and non-therapeutic method of the invention can be produced by the *Clostridia* bacteria and purified, or alternatively produced by recombinant or synthetic techniques. For example, the toxin may be a recombinant peptide, a fusion protein, or a hybrid neurotoxin, as prepared from subunits or domains of different botulinum toxin serotypes *(see, e.g.,* U.S. patent 6,444,209).

All seven botulinum toxin serotypes (A, B, C, D, E, F, and G) are commercially available from Sigma-Aldrich and from Metabiologics, Inc. (Madison, Wisconsin), as well as from other sources. At least two types of botulinum toxin, types A and B, are available commercially in formulations for treatment of certain human conditions. Type A, for example, is contained in BOTOX® (Allergan) and DYSPORT® (Ipsen), and Type B is contained in MYOBLOC® (Elan).

The botulinum toxin may alternatively be a botulinum toxin derivative, *e.g.,* a compound that has botulinum toxin activity but contains one or more chemical or functional alterations relative to naturally occurring or recombinant native botulinum toxins. For instance, the botulinum toxin can be a modified neurotoxin, e.g., a neurotoxin which has at least one of its amino acids deleted, modified or replaced, as compared to a native, or a recombinantly produced neurotoxin. For example, the botulinum toxin may be a Type A (including type A2 or Type A3) or Type B toxin having from 1 to about 20, or from 1 to about 10, or from 1 to about 5, amino acid substitutions, deletions, and/or insertions (collectively). The botulinum toxin may be a portion of the overall molecule that possesses the necessary botulinum toxin activity, and in such case may be used per se or as part of a combination or conjugate molecule, for instance a fusion protein.

In some embodiments, the botulinum toxin is a combination of botulinum toxin serotypes. For example, the invention may employ a combination of botulinum toxin serotype A and serotype B.

### Carrier Molecules

In accordance with the present invention, botulinum toxin is formulated with a carrier for effectively transporting the botulinum toxin to the cutaneous microvasculature. The invention allows for effective amounts of botulinum toxin to be controllably applied to affected regions of the skin (which may be large areas), and controllably delivered to the underlying microvasculature. In accordance with the invention, the carrier is a positively charged peptide carrier selected from Arg-Lys-Lys Arg-Arg-Gln-Arg-Arg-Arg-Gly-(Lys)n-Gly-Arg-Lys-Lys-Arg-Arg-Gln-Arg-Arg-Arg (SEQ ID NO:3); Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO:4), where n is an integer from 5 to 20; and Arg-Lys-Lys-Arg-Arg-Gln-Arg-Arg-Arg-Gly-(Lys) 15-Gly-Arg-Lys-Lys-Arg-Arg-Gln-Arg-Arg-Arg (SEQ ID NO:5), for transporting the botulinum neurotoxin to the cutaneous microvasculature.

The carrier may comprise at least one protein transduction domain or transport domain, such as an HIV-TAT or reverse HIV-TAT amino acid sequence, as described in WO 2008/082885. Exemplary transport domains have an amino acid sequence that corresponds to the reverse sequence of the HIV-TAT basic region (amino acids 49-57 of naturally-occurring HIV-TAT protein). The reverse sequence of the HIV-TAT basic region (RRRQRRKKR (SEQ ID NO: 1)) is hereafter referred to as "reverse HIV-TAT."

Exemplary carriers thus include the peptides described in WO 2009/015385. For example, the carrier may be a cationic peptide that comprises an HIV-TAT sequence or reverse HIV-TAT sequence at the N- or C-terminus, or both the N-terminus and the C-terminus. For example, the peptide carrier may have an HIV-TAT sequence, such as Arg-Lys-Lys-Arg-Arg-Gln-Arg-Arg-Arg (SEQ ID NO:2), or a reverse HIV TAT sequence, such as Arg-Arg-Arg-Gln-Arg-Arg-Lys- Lys-Arg (SEQ ID NO:1), at the N- or C-terminus or both the N-terminus and the C-terminus.

The carrier may comprise an N-terminal portion that is an HIV-TAT or reverse HIV-TAT sequence, a C-terminal portion that is an HIV-TAT or reverse HIV-TAT sequence, and one or more cationic residues (e.g., Lys or Arg) between the N- terminal portion and the C-terminal portion. For example, the peptide may have from 5 to 20 cationic residues such as Lys between the N-terminal portion and the C-terminal portion, such as about 12, about 15, or about 17 cationic residues. Exemplary carriers thus include Arg-Lys-Lys-Arg-Arg-Gln-Arg-Arg-Arg-Gly-(Lys)n-Gly-Arg-Lys-Lys-Arg-Arg-Gln-Arg- Arg-Arg (SEQ ID NO:3), or Arg-Arg-Arg-Gln-Arg-Arg-Lys-Lys-Arg-Gly-(Lys)n- Gly-Arg- Arg-Arg-Gln-Arg-Arg-Lys-Lys-Arg (SEQ ID NO:4), where n is from about 5 to about 20, such as from about 10 to about 20. In one embodiment, the N-terminal portion of the peptide is an HIV-TAT sequence and the C-terminal portion of the peptide is an HIV-TAT sequence. Alternatively, or in addition, the carrier may comprise peptides where the N-terminal portion is a reverse HIV-TAT sequence and the C-terminal portion is a reverse HIV-TAT sequence. For example, the peptide may have the following amino acid sequence: Arg-Lys-Lys-Arg-Arg-Gln-Arg-Arg-Arg-Gly-(Lys) 15-Gly-Arg-Lys-Lys-Arg-Arg-Gln-Arg-Arg-Arg (SEQ ID NO:5).

Generally, the carrier is characterized as having a particularly high content of Arg and Lys residues. For example, the carrier may contain at least about 50%, collectively, of Arg and Lys amino acid residues, but may contain at least about 75%, or at least about 80%, Arg and Lys residues. In these or other aspects of the disclosure, such derivatives may have the amino acid sequence of SEQ ID NO: 3, 4, or 5 with from 1 to 5 amino acid substitutions, insertions, or deletions (collectively), including 1, 2, 3, or 4 ammo acid substitutions, insertions, or deletions with respect to SEQ ID NO: 3, 4, or 5. In certain aspects of the disclosure, such substitutions, insertions, or deletions, are located within the HIV-TAT or reverse HIV-TAT sequence.

The carrier may be a peptide having a length of from about 15 amino acids to about 100 amino acids. In certain aspects, the carrier is from about 25 to about 50, or from about 25 to about 40 amino acids in length. In an exemplary embodiment of the invention, the carrier peptide is about 35 amino acids in length. The length of the carrier can be designed to carry botulinum neurotoxin to different depths of penetration in the skin by varying the length of the carrier. For example, longer polymeric carriers will penetrate to deeper layers of the skin.

Other disclosed carriers suitable for use in connection with the invention include a positively charged polymeric carrier to deliver the botulinum toxin across the skin barrier to desired depths of the underlying dermis. Such carriers include the polymeric carriers disclosed in U.S. Patent Publication No. 2004/0220100 and WO 2005/084410. In these aspects, the positively charged polymeric carrier comprises positively charged branching groups attached to a polymeric backbone. As used herein, "positively charged" means that the carrier has a positive charge under physiologically compatible conditions.

Generally, the positively-charged polymeric carrier is a linear chain of atoms, either with groups in the chain carrying a positive charge at physiological pH, or with groups carrying a positive charge attached to side chains extending from the backbone. The positively charged backbone itself need not have a defined enzymatic or therapeutic biologic activity. The linear backbone is a hydrocarbon backbone which is, in some embodiments, interrupted by heteroatoms selected from nitrogen, oxygen, sulfur, silicon and phosphorus. The majority of backbone chain atoms are carbon. Additionally, the backbone can be a polymer of repeating units *(e.g.,* amino acids, poly(ethyleneoxy), poly(propyleneamine), polyalkyleneimine, and the like), but may alternatively be a heteropolymer. In some aspects of the disclosure, the polymeric backbone is a positively charged polypeptide (e.g., polylysine). In other aspects, the positively charged backbone is a polypropyleneamine wherein a number of the amine nitrogen atoms are present as ammonium groups (tetra-substituted) carrying a positive charge.

The positively charged backbone may be a nonpeptidyl polymer, which may be a hetero- or homo-polymer such as a polyalkyleneimine, for example a polyethyleneimine or polypropyleneimine, having a molecular weight of from about 10,000 to about 2,500,000, or from about 100,000 to about 1,800,000, or from about 500,000 to about 1,400,000. The length of the polymeric carrier can be designed to carry botulinum neurotoxin to different depths of penetration in the skin by varying the length of the carrier. For example, longer polymeric carriers will penetrate to deeper layers of the skin.

In certain aspects of the disclosure, the backbone has attached a plurality of side-chain moieties that include positively charged groups (*e.g.,* ammonium groups, pyridinium groups, phosphonium groups, sulfonium groups, guanidinium groups, or amidinium groups). The sidechain moieties may be spaced along the backbone in a consistent or variable manner. Additionally, the length of the sidechains can be similar or dissimilar. For example, in some aspects, the sidechains can be linear or branched hydrocarbon chains having from one to twenty carbon atoms and terminating at the distal end (away from the backbone) in one of the above-noted positively charged groups.

The association between the carrier and the biologically active agent (*e.g.* botulinum toxin) is generally by non-covalent interaction, such as by ionic interactions, hydrogen bonding, van der Waals forces, or combinations thereof.

In some aspects of the disclosure, the positively charged backbone is a polypeptide having multiple positively charged sidechain groups (*e.g.*, lysine, arginine, omithine, homoarginine, and the like). Preferably, the polypeptide has a molecular weight of from about 10,000 to about 1,500,000, or from about 25,000 to about 1,200,000, or from about 100,000 to about 1,000,000. One of skill in the art will appreciate that when amino acids are used in the carrier, the sidechains can have either the D- or L-form (R or S configuration) at the center of attachment. Alternatively, the backbone can be an analog of a polypeptide such as a peptoid. See, for example, Kessler, Angew. Chem. Int. Ed. Engl., Vol. 32:543 (1993); Zuckermann et al. Chemtracts-Macromol. Chem., Vol. 4:80 (1992); and Simon et al. Proc. Nat'l. Acad. Sci. USA, Vol. 89:9367 (1992). Briefly, a peptoid is a polyglycine in which the sidechain is attached to the backbone nitrogen atoms rather than the a-carbon atoms. A portion of the sidechains can terminate in a positively charged group to provide a positively charged backbone component. Synthesis of peptoids is described in, for example, U.S. Patent No. 5,877,278. As the term is used herein, positively charged backbones that have a peptoid backbone construction are considered "non-peptide" as they are not composed of amino acids having naturally occurring sidechains at the a-carbon locations.

A variety of other backbones can be used employing, for example, steric or electronic mimics of polypeptides wherein the amide linkages of the peptide are replaced with surrogates such as ester linkages, thioamides (-CSNH-), reversed thioamide (-NHCS-), aminomethylene (-NHCH₂-) or the reversed methyleneamino(-CH2NH-) groups, ketomethylene (-COCH₂-) groups, phosphinate (-PO₂RCH₂-), phosphonamidate and phosphonamidate ester (-PO₂RNH-), reverse peptide (-NHCO-), trans-alkene (-CR=CH-), fluoroalkene (-CF=CH-), dimethylene (-CH₂CH₂-), thioether (-CH₂S-), hydroxyethylene (-CH(OH)CH₂-), methyleneoxy (-CH₂O-), tetrazole (CN₄), sulfonamido (-SO₂NH-), methylenesulfonamido (-CHRSO₂NH-), reversed sulfonamide (-NHSO₂-), and backbones with malonate and/or gem-diamino-alkyl subunits, for example, as reviewed by Fletcher et al. ((1998) Chem. Rev., Vol. 98:763) and detailed by references cited therein. Many of the foregoing substitutions result in approximately isosteric polymer backbones relative to backbones formed from α-amino acids.

In each of the backbones provided above, sidechain groups can be appended that carry a positively charged group. For example, the sulfonamide-linked backbones (-SO₂NH- and -NHSO₂-) can have sidechain groups attached to the nitrogen atoms. Similarly, the hydroxyethylene (-CH(OH)CH₂) linkage can bear a sidechain group attached to the hydroxy substituent. One of skill in the art can readily adapt the other linkage chemistries to provide positively charged sidechain groups using standard synthetic methods.

In one aspect, the positively charged polymeric carrier is a polypeptide having branching groups (also referred to as efficiency groups). As used herein, an efficiency group or branching group is any agent that has the effect of promoting the translocation of the positively charged polymeric carrier through a tissue or cell membrane. Non-limiting examples of branching or efficiency groups include HIV-TAT or reverse HIV-TAT (as described) or fragments thereof, the protein transduction domain of Antennapedia, or a fragment thereof, or -(gly)ₙ₁-(arg)ₙ₂, wherein n1 is an integer of from about Oto about 20, or 0 to about 8, or 2 to about 5. n2 is independently an odd integer of from about 5 to about 25, or about 7 to about 17, or about 7 to about 13.

Other aspects of the disclosure employ HIV-TAT fragment or reverse HIV-TAT sequences having the formula (gly)ₚ-RGRDDRRQRRR-(gly)_{q} (SEQ ID NO: 6), (gly)ₚ-YGRKKRRQRRR-(gly)_{q} (SEQ ID NO: 7), or (gly)ₚ-RKKRRQRRR-(gly)_{q} (SEQ ID NO: 8) when in p and q are each independently an integer of from 0 to 20 and the fragment is attached to the backbone via either the C-terminus or the N-terminus of the fragment. HIV-TAT fragments include those in which p and q are each independently integers of from Oto about 8, such as from 2 to about 5.

The positively charged side chain or branching group may be the Antennapedia (Antp) protein transduction domain (PTD), or a fragment thereof that retains activity. These are known in the art, for instance, from Console et al., J Biol. Chem., Vol. 278:35109 (2003).

The positively charged polymeric earner may include positively charged branching groups in an amount of at least about 0.05%, as a percentage of the total carrier weight, such as from about 0.05 to about 45%, or about 0.1 to about 30%. For positively charged branching groups having the formula-(gly)ₙ₁(arg)ₙ₂, the amount may be from about 0.1 to about 25 %.

In certain aspects, the backbone portion of the carrier is a polylysine and positively charged branching groups (as described above) are attached to the lysine sidechain amino groups. The polylysine may have a molecular weight of from about 10,000 to about 1,500,000, such as from about 25,000 to about 1,200,000, or from about 100,000 to about 1,000,000. It can be any of the commercially available polylysines (Sigma Chemical Company, St. Louis, Missouri, USA) such as, for example, polylysine having MW > 70,000, polylysine having MW of 70,000 to 150,000, polylysine having MW 150,000 to 300,000 and polylysine having MW > 300,000. The selection of an appropriate polylysine will depend on the remaining components of the composition and will be sufficient to provide an overall net positive charge to the composition and provide a length that is preferably from one to four times the combined length of the negatively charged components.

In other aspects, the carrier is a relatively short polylysine or polyethyleneimine (PEI) backbone (which may be linear or branched) and which has positively charged branching groups. Such carriers may be useful for minimizing uncontrolled aggregation of the backbones and botulinum toxin in a therapeutic composition, which causes the transport efficiency to decrease dramatically. When the carrier is a relatively short linear polylysine or PEI backbone, the backbone will have a molecular weight of less than about 75,000, or less than about 30,000, or less than about 25,000. When the carrier is a relatively short branched polylysine or PEI backbone, however, the backbone will have a molecular weight less than about 60,000, or less than about 55,000, or less than about 50,000. If, however, partitioning agents as described herein are included in the composition, the molecular weight of the branched polylysine and PEI backbones can be up to about 75,000, while the molecular weight of the linear polylysine and PEI backbones can be up to about 150,000.

### Compositions and Formulations

The compositions of the invention preferably comprise an effective amount of a botulinum toxin. An "effective amount" is an amount sufficient to affect a beneficial or desired clinical result, *e.g.* an amount sufficient to reduce vascular hyper-reactivity in the skin. An effective amount in each embodiment will be based on the serotype of botulinum toxin, the form of toxin used (*e.g*. complex or purified di-chains), and in some embodiments, factors individual to the patient, including age, condition to be treated (*e.g*. flushing, blushing, particular subtype of rosacea), size of skin area to be treated, and severity of symptoms.

In some embodiments, an effective amount is from about 0.1 ng to about 2.0 ng of purified botulinum neurotoxin (e.g., serotype A). The effective amount may be from about 0.2 ng to about 1.5 ng, or from about 0.5 ng to about 1.0 ng of purified botulinum neurotoxin (e.g., serotype A or botulinum neurotoxin Type A complex). The effective amount may be combined in about 0.5 ml to about 10 mls of cream or gel, as described herein, depending on the size of the area needing treatment.

Compositions for use in the methods of the invention are formulated for application to the skin or epithelium of subjects or patients, *i.e*. humans or other mammals in need of the particular treatment. In general, the compositions are prepared by mixing the botulinum toxin with the carrier (described above), and optionally with one or more additional pharmaceutically acceptable diluents or excipients. The ratio of carrier to botulinum toxin may be in the range of about 10:1 to about 1:10 by mass (e.g., about 1:1). The ratio may be from about 6:1 to about 1.5:1 respectively. In such embodiments, the carrier is the peptide of SEQ ID NO: 3, 4, or 5.

The compositions may contain other ingredients typical m topical pharmaceutical or cosmeceutical compositions, including a dermatologically or pharmaceutically acceptable carrier, vehicle or medium, *(i.e.* a carrier, vehicle or medium that is compatible with the tissues to which they will be applied). The term "dermatologically or pharmaceutically acceptable," as used herein, means that the compositions or components thereof so described are suitable for use in contact with these tissues or for use in patients in general without undue toxicity, incompatibility, instability, allergic response, and the like. As appropriate, compositions suitable for use in the methods of the invention can comprise any ingredient conventionally used in the fields under consideration, and particularly in cosmetics and dermatology. The compositions can also include a quantity of a small anion, preferably a polyvalent anion, for example, phosphate, aspartate, orcitrate.

In terms of their form, the topical botulinum toxin compositions can include solutions, emulsions (including microemulsions), suspensions, creams, lotions, gels, powders, ointments or other typical solid or liquid compositions used for application to skin and other tissues where the compositions may be used. Such compositions may contain, in addition to the botulinum toxin and carrier, other ingredients typically used in such products, such as antimicrobials, moisturizers and hydration agents, penetration agents, preservatives, emulsifiers, natural or synthetic oils, solvents, surfactants, detergents, emollients, antioxidants, fragrances, fillers, thickeners, waxes, odor absorbers, dyestuffs, coloring agents, powders, and optionally include anesthetics, anti-itch additives, botanical extracts, conditioning agents, darkening or lightening agents, glitter, humectants, mica, minerals, polyphenols, silicones or derivatives thereof, sunblocks, vitamins, and phytomedicinals.

In certain embodiments, the compositions include gelling agents and/or viscosity-modifying agents. These agents are generally added to increase the viscosity of the composition, so as to make the application of the composition easier and more accurate. Additionally, these agents help to prevent the aqueous botulinum toxin/carrier solution from drying out, which tends to cause a decrease in the activity of the botulinum toxin. Exemplary agents are those that are uncharged and do not interfere with the botulinum toxin activity or the efficiency of the toxin-carrier complexes in crossing skin. The gelling agents may be certain cellulose-based gelling agents, such as hydroxypropylcellulose (HPC) for example. In some embodiments, the botulinum toxin/carrier complex is formulated in a composition having 2-4% HPC. Alternatively, the viscosity of a solution containing a botulinum toxin/carrier complex may be altered by adding polyethylene glycol (PEG). In other embodiments, the botulinum toxin/carrier solution IS combined with pre-mixed viscous agents, such as Cetaphil® moisturizer.

In certain embodiments, the composition further comprises a poloxamer-based diluent. The poloxamer diluent is a copolymer surfactant amenable to diluting lyophilized protein, and then gelling to a viscosity sufficient to be applied and remain in a defined cutaneous area. The poloxamer may be present at about 10% to 20%, for example, in 0.9% saline. At about 15%, the poloxamer is thermal-sensitive. That is, the composition will remain liquid at temperatures of 20°C or less, with a gel product forming at temperatures in the range of 20-35°C for local application.

The botulinum toxin compositions can optionally include partitioning agents. As used herein, a "partitioning agent" is any substance or additive that has the property of preventing or minimizing unwanted or uncontrolled aggregation of the botulinum toxin with the positively charged polymeric carriers. Partitioning agents are particularly useful, for example, when a concentrated botulinum toxin solution is employed due to volume constraints. In such embodiments, the partitioning agent keeps the botulinum toxin dispersed, thereby preventing aggregation of the toxin that would otherwise occur without the partitioning agent. Generally, a partitioning agent is (1) non-irritating, (2) does not destroy the botulinum toxin, (3) does not confer any increase in permeability, (4) affords reliable and stable particle sizes, (5) is uncharged, and (6) does not interfere with complexes of the toxin and the polymeric carrier.

An example of a suitable partitioning agent is ethanol (EtOH). For example, the EtOH may be present at less than 20% of the composition, such as less than about 5% of the composition. By way of example, if volume constraints require reconstituting 100 U of botulinum toxin in 0.5 ml of solution, rather than 2.5 ml, one typically observes that the botulinum toxin will exhibit undesirable aggregation, and thus lowered activity. However, by adding 1% EtOH as a dispersing agent, full activity is maintained even after 24 hours at this concentration. As another example, Botox® at 1.0 ml 0.9% NaCl reconstitution has full activity, while reconstitution at 0.5 ml in 1% and 5% EtOH plus 0.9% NaCl produces solutions with full activity.

In certain embodiments of the invention, oligo- or polyanion bridges are added to the botulinum toxin compositions to improve the complexation of the toxin with a positively charged carrier. Such bridges are particularly useful when the botulinum toxin complexes are used in the compositions. Some of the complex proteins are positively charged, and others are negatively charged. Because the exact distribution of the components of the toxin varies depending on the source of the toxin, it may be that botulinum toxin from certain sources has a lower propensity for complexation with the positively charged polymeric carriers described herein. However, by adding an oligo- or polyanion bridge to such botulinum toxin complexes, the efficiency and efficacy of topical administration is increased dramatically. Suitable examples of such oligo-/polyanion bridges include sodium phosphate (5%), PBS or 5% poly-L-aspartate (*e.g*., with a MW of 3000).

Compositions according to this invention can be in the form of controlled-release or sustained-release compositions, wherein the botulinum toxin and the carrier are encapsulated or otherwise contained within a material such that they are released onto the skin in a controlled manner over time. The botulinum toxin and carrier may be contained within matrixes, liposomes, vesicles, microcapsules, microspheres and the like, or within a solid particulate material, all of which is selected and/or constructed to provide release of the botulinum toxin over time. The botulinum toxin and the carrier may be encapsulated together *(e.g.,* in the same capsule) or separately (in separate capsules).

### Application of Topical Composition

The compositions for use in a method of treatment, as according to the invention, are administered by or under the direction of a physician or other health care professional. The compositions can be administered in a single treatment or in a series of periodic treatments over time. It is expected that the effect of the treatment will last for about 3, 4, 5, or 6 months. Thus, the topical composition may be administered once, or may be administered repeatedly, or routinely. The frequency of the treatment can vary, but may be, for example, once a month, or two to ten times per year, such as two to five times per year. Because the treatment is non-irritating and does not exacerbate the underlying condition, long term treatment for 1, 2, 3, or 4 or more years is possible if necessary.

For topical delivery of botulinum toxin according to the methods as described herein, a composition as described above is applied topically to the skin at a location or locations where the effect is desired (*e.g*. affected areas of the skin). In embodiments where an aqueous botulinum toxin/carrier solution is to be applied directly to the skin, it is preferable to cover the treated area (e.g., with Cetaphil® moisturizer) or occlude the treated area with a barrier (e.g., Telfa), in order to prevent the solution from drying out, which would lead to a decrease in toxin activity. Because of its nature, most preferably the amount of botulinum toxin applied should be applied with care, at an application rate and frequency of application that will produce the desired result without producing any adverse or undesired results. Accordingly, for instance, topical compositions of the invention should be applied at a rate of from about 1U to about 20,000U, preferably from about 1U to about 10,000U botulinum toxin per cm² of skin surface. Higher dosages within these ranges could preferably be employed in conjunction with controlled release materials (*e.g*. patches and the like), for instance, or allowed a shorter dwell time on the skin prior to removal.

In some embodiments, the method for treating a patient having a skin condition characterized by vascular hyper-reactivity further comprises preparing the skin surface prior to application of the topical botulinum toxin composition. In certain embodiments, preparation of the skin surface prior to the application of the botulinum toxin/carrier composition promotes the efficacy of the solution. For example, the introduction of surfactants on the surface of the skin for the purpose of cleaning off surface oils on the skin prior to application may be counterproductive, because the surfactants appear to destroy the activity of the botulinum toxin. This occurs even if the skin is subsequently washed with water several times before application of the botulinum toxin/carrier solution. Even extremely gentle surfactants, such as those found in baby wipes, appear to cause this phenomenon. Accordingly, in certain embodiments, the skin is pre-cleaned using water alone. Washing with only water also appears to improve the transdermal transport of the botulinum toxin moderately.

### Kits and Delivery Devices

The present invention provides transdermal devices for treating a patient having a skin condition characterized by vascular hyper-reactivity. Such devices contain botulinum toxin and a carrier as described herein. Such devices can be as simple in construction as a skin patch, or may be more complicated devices that include means for dispensing and monitoring the dispensing of the composition, and optionally means for monitoring the condition of the subject (*e.g.*, monitoring the reaction of the subject to the substances being dispensed). In one embodiment, the present invention provides a skin patch comprising an effective amount of a botulinum toxin and a carrier as described.

The materials for the construction of delivery devices may be those that do not lead to a loss of activity of the botulinum toxin/carrier solution, either through degradation or unwanted adsorption of the botulinum toxin on a surface of the device. Such undesired behavior has been observed, for example, when botulinum toxin/carrier in an aqueous solution contacts polypropylene surfaces, but not when the botulinum toxin/carrier solution contacts polyvinyl chloride (PVC) surfaces.

Generally, the compositions can be pre-formulated and/or pre-installed in a device or can be prepared later, for example using a kit comprising the two ingredients (botulinum toxin and carrier) separately, but providing a mechanism for combining them at or prior to the time of application. By "in conjunction with" is meant that the two components (botulinum toxin and carrier) are administered in a combination procedure, which may involve either combining them in a composition, which is subsequently administered to the subject, or administering them separately, but in a manner such that they act together to provide the requisite delivery of an effective amount of the botulinum toxin.

For example, a composition containing the carrier may first be applied to the skin of the patient, followed by applying a skin patch or other device containing the botulinum toxin. The botulinum toxin can be incorporated in dry form in a skin patch or other dispensing device, while the positively charged polymeric carrier can be applied to the skin surface in a cream or lotion before application of the patch so that the two act together, resulting in the desired transdermal delivery. Thus, the two substances (carrier and botulinum toxin) act in combination or can interact to form a composition or combination *in situ.*

Thus, the present invention also provides a kit for treating a skin condition characterized by vascular hyper-reactivity. The kit comprises an effective amount of a botulinum toxin, a carrier, and a diluent, such as a poloxamer diluent as described herein. In one embodiment, the skin condition is rosacea. In some aspects, the botulinum toxin is packaged separately from the other components in the kit. For instance, the botulinum toxin can be lyophilized and stored in a separate vial than the carrier and/or diluent. In another embodiment, the botulinum toxin is packaged within a device, *e.g.* a skin patch.

In yet another aspect, the positively charged polymeric carrier is pre-formulated in a liquid, gel, cream, or the like for application to the skin or epithelium of a patient. In certain embodiments, the kit can further comprise utensils and/or an applicator for mixing the botulinum toxin with the positively charged polymeric carrier and diluent and applying the topical mixture to an affected area of the patient's skin.

In other embodiments, the kit can comprise components for maintaining the treated area between or after neurotoxin treatments. For example, the kit may comprise a gentle skin cleanser(s) for removing the excess botulinum toxin composition following treatment, a redness reducing lotion, one or more additional topical treatments for rosacea, and/or sun protection products with an SPF of 15 or greater.

For example, between in-office treatments, patients may be provided with the after topical neurotoxin treatment system or kit for maintaining the treated area. The kit comprises a gentle skin cleanser (which the patient may apply in the morning and/or evening), a topical redness reducing lotion (for example, Eucerin® Anti-Redness system or other lotions containing licorice), which may be applied after the gentle skin cleanser, and a broad spectrum sunblock of SPF 15 or greater (to protect the treated area during the day). In the evening the patient may wash again with the gentle cleanser, followed by topical administration of one or more topical rosacea treatments, such as, for example, metronidazole gel (USP ranging from 0.75% to 1.0%).

The following examples are included to further illustrate various aspects of the invention.

### EXAMPLES

### Example 1: Topical formulation of botulinum neurotoxin

An exemplary formulation comprises: a purified 150 kDa botulinum toxin type A, which is minimally immunogenic and contains no animal or human components; the carrier peptide (R-K-K-R-R-Q-R-R-R-G-(K)₁₅-G-R-K-K-R-R-Q-R-R-RSEQ ID NO:5), which enables the transcutaneous delivery of the botulinum toxin to the underlying dermis; and a poloxamer-based diluent, which acts as a vehicle for the carrier.

The botulinum toxin is produced by *Clostridium botulinum* (serotype A - Hall strain) in a fermentation culture, and the fully active di-chain comprised of a 100-kDa heavy chain and a 50-kDa light chain is purified from the culture medium. Unlike the commercially available BOTOX® Cosmetic (Allergan), the purified 150 kDa neurotoxin is not associated with any of the bacterially-derived accessory proteins or formulated in human serum albumin, hemaglutinin, or other pooled human-derived components.

The purified botulinum neurotoxin is combined with the peptide carrier in a ratio of about 1:1 by mass, and diluted with phosphate buffered saline to 200 µL. The resulting mixture is combined with 1.8 mL of poloxamer-based diluent (e.g., Pluronic® Fl 27, NF Grade, BASF Corp.). The poloxamer diluent is a copolymer surfactant amenable to diluting lyophilized protein, and then gelling to a viscosity sufficient to be applied and remain in a defined cutaneous area. At 15% (qs with 0.9% saline), the poloxamer is thermal-sensitive. That is, the composition will remain liquid at temperatures of 20°C or less, with a gel product forming at temperatures in the range of 20-35°C for local application.

### Example 2: Treatment of facial flushing with a topical botulinum neurotoxin composition

For treatment of facial flushing, a vial containing 1 nanogram of lyophilized botulinum neurotoxin type A is mixed with a second vial containing a liquid diluent containing peptide carrier and the poloxamer-based diluent (see Example 1). The components are mixed together to form a smooth viscous gel that is applied topically to affected areas (e.g., a patient's cheeks and nasal fold areas).

It is expected that within 2 weeks of treatment, the patient will note significant improvement of, for example, facial erythema at rest and at exercise. For example, after treatment, flushing will be only slightly perceptible in the general treatment areas. Small telangiectasias will only be slightly perceptible after treatment with the topical botulinum neurotoxin gel. It is also expected that there are no visible signs of cheek droop, which is a reported adverse event when using injectable botulinum neurotoxin. Results are expected to persist for 2 months ormore.

### Example 3: Treatment of rosacea with a topical botulinum neurotoxin composition

For treatment of rosacea, including with moderate swelling, erythema and papules of the central part of the face, 1.2 nanograms of lyophilized botulinum neurotoxin type A is mixed with the carrier peptide and the poloxamer-based diluent (Example 1). The composition is applied topically to all the affected areas of the patient's central face.

It is expected that by about 3 days after treatment, the skin condition will rapidly improve. A second application of the topical botulinum neurotoxin gel may then be applied at about four weeks, and the condition of the skin will continue to improve. Four months after the second treatment, it is expected that the patient will remain almost clear of rosacea symptoms (dilated blood vessels, papules).

### REFERENCES

(1) Jacobson MJ, Guangyun L, Raphael B, Andreadis J, Johnson EA, Analysis ofNeurotoxin Cluster Genes in Clostridium botulinum Strains Producing Botulinum Neurotoxin Serotype A Subtypes, Appl. Environ. Microbial. 74(9):2778-2786 (2008).
(2) Alexandroff AB, Sinclair SA, Langtry JA, Successful use of botulinum toxin type A for the treatment of neck and anterior chest wall flushing. Derm. Surg. 32(12): 1536-1536 (2006).
(3) Bull HA, Hothersall J, Chowdhury N, Cohen J, Dowd P, Neuropeptides induce release of nitric oxide from human dermal microvascular endothelial cells. Journal of Invest. Dermatol. 106:655-660(1996).
(4) Carruthers J, Carruthers A, The effect of full-face broadband light treatments alone and in combination with bilateral crow's feet botulinum toxin type a chemodenevation. Dermagol Surg 30:355-366 (2004).
(5) Cohen-Arad A, Blitzer A, Botulinum toxin treatment for symptomatic Frey's syndrome. Otolaryngology-Head and Neck Surg 122: 237-240 (2000).
(6) Jacob Cl. Dermatologic Surgery. 31(4): 492-492 (March 2006).
(7) Kranendonk, SK, Re: Botulinum Toxin for the Treatment of Facial Flushing. Dermatologic Surgery. 31(4):491-491 (March 2006).
(8) Lowe N, Campanati A, Bodokh I, Cliff S, Jaen P, Kreyden 0, Naumann M, Offidani A, Vadoud J, and Hamm H, The place of botulinum toxin type A in the treatment of focal hyperhidrosis. British Journal of Dermatology 151: 1115-1122 (2004).
(9) Sato K, Kang WH, Saga Kand Sato KT, Biology of sweat glands and their disorders. II Disorders of sweat gland function. JAm Acad Dermatol 20:713-726 (1989).

## Claims

1. A topical composition for use in a method of treating a patient having a skin condition **characterized by** cutaneous vascular hyper-reactivity,
wherein the topical composition is applied to area(s) of the patient's skin affected with said vascular hyper-reactivity, and
wherein the topical composition comprises an amount of a botulinum neurotoxin effective for decreasing vasodilation in the cutaneous microvasculature,
wherein said botulinum neurotoxin is non-covalently associated with a positively charged peptide carrier selected from Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID NO:3); Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO:4), where n is an integer from 5 to 20; and Arg-Lys-Lys-Arg-Arg-Gln-Arg-Arg-Arg-Gly-(Lys)15-Gly-Arg-Lys-Lys-Arg-Arg-Gln-Arg-Arg-Arg (SEQ ID NO:5), for transporting the botulinum neurotoxin to the cutaneous microvasculature.

2. The topical composition for use according to claim 1, wherein the skin condition is one or more selected from flushing, blushing, and rosacea.

3. The topical composition for use according to claim 2, wherein said rosacea is of a type selected from one or more of erythematotelangiectatic rosacea, papulopustular rosacea, phymatous rosacea, and ocular rosacea.

4. The topical composition for use according to any one of claims 1 to 3, wherein the botulinum neurotoxin is a serotype selected from botulinum neurotoxin serotypes A, B, C, D, E, F, or G.

5. The topical composition for use according to claim 4, wherein the botulinum neurotoxin is serotype A.

6. The topical composition for use according to claim 5, wherein the botulinum neurotoxin is derived from the Hall Strain.

7. The topical composition for use according to claim 1, wherein the botulinum neurotoxin is part of a complex having a molecular weight ranging from 450 kDa to 900 kDa.

8. The topical composition for use according to claim 5, wherein the botulinum neurotoxin is purified and has a molecular weight of 150 kDa.

9. The topical composition for use according to claim 8, wherein the effective amount of botulinum neurotoxin is from 0.1 ng to 2.0 ng of purified botulinum neurotoxin per 0.5 mL to 10 mL of cream or gel.

10. The topical composition for use according to claim 8, wherein the effective amount of botulinum neurotoxin is from 1 U to about 20,000 U botulinum toxin per cm² of skin surface.

11. The topical composition for use according to claim 10, wherein the effective amount of botulinum neurotoxin is from 1 U to about 10,000 U botulinum toxin per cm² of skin surface.

12. The topical composition for use according to claim 7 or 8, wherein the positively charged peptide carrier is selected from Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID NO:3); and Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO:4), where n is an integer from 10 to 20.

13. The topical composition for use according to claim 7 or 8, wherein the positively charged peptide carrier is Arg-Lys-Lys-Arg-Arg-Gln-Arg-Arg-Arg-Gly-(Lys)₁₅-Gly-Arg-Lys-Lys-Arg-Arg-Gln-Arg-Arg-Arg (SEQ ID NO: 5).

14. The topical composition for use according to claim 12 or 13, wherein the composition further comprises a poloxamer-based diluent.

15. The topical composition for use according to claim 14, wherein the poloxamer-based diluent is present at 10% to 20% by volume.

16. The topical composition for use according to claim 15, wherein the poloxamer-based diluent is present at 15% by volume.

17. The topical composition for use according to any one of claims 1 to 16, wherein said botulinum neurotoxin in the composition is produced by recombinant technique.

18. The topical composition for use according to any one of claims 3 to 17, wherein said patient has rosacea but not rhinophyma.

19. A skin patch comprising the topical composition as defined in any one of claims 12 to 18 and a patch for the skin.

20. A kit for treating a skin condition **characterised by** vascular hyper-reactivity, which kit comprises a topical composition as defined in any one of claims 12 to 18, further comprising one or more components selected from a gentle skin cleanser(s) for removing the excess botulinum toxin composition following treatment, a redness reducing lotion, one or more additional topical treatments for rosacea, and sun protection products with an SPF of 15 or greater.

21. Use of a composition as defined in any one of claims 12 to 18, in the manufacture of a medicament for treating a patient having a skin condition **characterized by** cutaneous vascular hyper-reactivity.

22. A non-therapeutic method for treating a patient having a skin condition **characterized by** cutaneous vascular hyper-reactivity, comprising applying a topical composition to the affected area(s) of the patient's skin, wherein the topical composition is as defined in any one of claims 12 to 18.

## Patentansprüche

1. Topische Zusammensetzung zur Verwendung in einem Verfahren zum Behandeln eines Patienten, der einen Hautzustand aufweist, der durch kutane vaskuläre Hyperreaktivität gekennzeichnet ist,
wobei die topische Zusammensetzung auf einen Bereich/Bereiche der Haut des Patienten aufgetragen wird, der/die von der vaskulären Hyperreaktivität betroffen ist/sind, und
wobei die topische Zusammensetzung eine Menge eines Botulinum-Neurotoxins umfasst, das wirksam ist, um eine Vasodilatation in der kutanen Mikrovaskulatur zu reduzieren,
wobei das Botulinum-Neurotoxin nicht-kovalent mit einem positiv geladenen Peptidträger assoziiert ist, der ausgewählt ist aus Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID NO: 3); Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO: 4), wobei n eine Ganzzahl von 5 bis 20 ist; und Arg-Lys-Lys-Arg-Arg-Gln-Arg-Arg-Arg-Gly-(Lys) 15-Gly-Arg-Lys-Lys-Arg-Arg-Gln-Arg-Arg-Arg (SEQ ID NO: 5), zum Transportieren des Botulinum-Neurotoxins zu der kutanen Mikrovaskulatur.

2. Topische Zusammensetzung zur Verwendung nach Anspruch 1, wobei der Hautzustand einer oder mehrere ist, die aus Durchspülen, Erröten und Rosacea ausgewählt sind.

3. Topische Zusammensetzung zur Verwendung nach Anspruch 2, wobei die Rosacea von einem Typ ist, der ausgewählt ist aus einer oder mehreren von erythematotelangiektatischer Rosacea, papulopustulöser Rosacea, phymatischer Rosacea und okulärer Rosacea .

4. Topische Zusammensetzung zur Verwendung nach einem der Ansprüche 1 bis 3, wobei das Botulinum-Neurotoxin ein Serotyp ist, ausgewählt aus Botulinum-Neurotoxin-Serotypen A, B, C, D, E, F oder G.

5. Topische Zusammensetzung zur Verwendung nach Anspruch 4, wobei das Botulinum-Neurotoxin Serotyp A ist.

6. Topische Zusammensetzung zur Verwendung nach Anspruch 5, wobei das Botulinum-Neurotoxin von dem Hall-Stamm abgeleitet ist.

7. Topische Zusammensetzung zur Verwendung nach Anspruch 1, wobei das Botulinum-Neurotoxin Teil eines Komplexes ist, der ein Molekulargewicht in einem Bereich von 450 kDa bis 900 kDa aufweist.

8. Topische Zusammensetzung zur Verwendung nach Anspruch 5, wobei das Botulinum-Neurotoxin aufgereinigt ist und ein Molekulargewicht von 150 kDa aufweist.

9. Topische Zusammensetzung zur Verwendung nach Anspruch 8, wobei die wirksame Menge von Botulinum-Neurotoxin von 0,1 ng bis 2,0 ng aufgereinigtes Botulinum-Neurotoxin pro 0,5 ml bis 10 ml Creme oder Gel ist.

10. Topische Zusammensetzung zur Verwendung nach Anspruch 8, wobei die wirksame Menge von Botulinum-Neurotoxin von 1 U bis etwa 20.000 U Botulinumtoxin pro cm² Hautoberfläche ist.

11. Topische Zusammensetzung zur Verwendung nach Anspruch 10, wobei die wirksame Menge von Botulinum-Neurotoxin von 1 U bis etwa 10.000 U Botulinumtoxin pro cm² Hautoberfläche ist.

12. Topische Zusammensetzung zur Verwendung nach Anspruch 7 oder 8, wobei der positiv geladene Peptidträger ausgewählt ist aus Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID NO: 3); und Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID NO: 4), wobei n eine Ganzzahl von 10 bis 20 ist.

13. Topische Zusammensetzung zur Verwendung nach Anspruch 7 oder 8, wobei der positiv geladene Peptidträger Arg-Lys-Lys-Arg-Arg-Gln-Arg-Arg-Arg-Gly-(Lys)₁₅-Gly-Arg-Lys-Lys-Arg-Arg-Gln-Arg-Arg-Arg (SEQ ID NO: 5) ist.

14. Topische Zusammensetzung zur Verwendung nach Anspruch 12 oder 13, wobei die Zusammensetzung ferner ein auf Poloxamer basiertes Verdünnungsmittel umfasst.

15. Topische Zusammensetzung zur Verwendung nach Anspruch 14, wobei das auf Poloxamer basierte Verdünnungsmittel mit 10 % bis 20 % nach Volumen vorhanden ist.

16. Topische Zusammensetzung zur Verwendung nach Anspruch 15, wobei das auf Poloxamer basierte Verdünnungsmittel mit 15 % nach Volumen vorhanden ist.

17. Topische Zusammensetzung zur Verwendung nach einem der Ansprüche 1 bis 16, wobei das Botulinum-Neurotoxin in der Zusammensetzung durch rekombinante Technik erzeugt wird.

18. topische Zusammensetzung zur Verwendung nach einem der Ansprüche 3 bis 17, wobei der Patient Rosacea, aber nicht Rhinophym hat.

19. Hautpflaster, umfassend die topische Zusammensetzung wie definiert in einem der Ansprüche 12 bis 18 und ein Pflaster für die Haut.

20. Kit zum Behandeln eines Hautzustands, der durch vaskuläre Hyperreaktivität gekennzeichnet ist, wobei das Kit eine topische Zusammensetzung wie definiert in einem der Ansprüche 12 bis 18 umfasst, ferner umfassend eine oder mehrere Komponenten, die ausgewählt sind aus einem oder mehreren sanften Hautreinigungsmitteln zum Entfernen der überschüssigen Botulinumtoxin-Zusammensetzung nach Behandlung, einer rötungsverringernden Lotion, einer oder mehreren zusätzlichen topischen Behandlungen für Rosacea und Sonnenschutzprodukten mit einem SPF von 15 oder größer.

21. Verwendung einer Zusammensetzung wie definiert in einem der Ansprüche 12 bis 18 bei der Herstellung eines Medikaments zum Behandeln eines Patienten mit einem Hautzustand, der durch kutane vaskuläre Hyperreaktivität gekennzeichnet ist.

22. Nicht-therapeutisches Verfahren zum Behandeln eines Patienten mit einem Hautzustand, der durch kutane vaskuläre Hyperreaktivität gekennzeichnet ist, umfassend ein Auftragen einer topischen Zusammensetzung auf den/die betroffenen Bereich(e) der Haut des Patienten, wobei die topische Zusammensetzung wie definiert in einem der Ansprüche 12 bis 18 ist.

## Revendications

1. Composition topique pour une utilisation dans un procédé de traitement d'un patient atteint d'une affection de la peau **caractérisée par** une hyperréactivité vasculaire cutanée,
la composition topique étant appliquée sur au moins une zone de la peau du patient atteint de ladite hyperréactivité vasculaire, et
la composition topique comprenant une quantité de neurotoxine botulique efficace pour diminuer une vasodilatation de la microvasculature cutanée,
ladite neurotoxine botulique étant associée de façon non covalente à un support peptidique chargé positivement choisi parmi Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID N° : 3) ; Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID N° : 4), où n est un entier de 5 à 20 ; et Arg-Lys-Lys-Arg-Arg-Gln-Arg-Arg-Arg-Gly-(Lys)15-Gly-Arg-Lys-Lys-Arg-Arg-Gln-Arg-Arg-Arg (SEQ ID N° : 5), pour transporter la neurotoxine botulique jusqu'à la microvasculature cutanée.

2. Composition topique pour une utilisation selon la revendication 1, dans laquelle l'affection de la peau est au moins l'une choisie parmi les rougeurs, le rougissement et la rosacée.

3. Composition topique pour une utilisation selon la revendication 2, dans laquelle ladite rosacée est d'un type choisi parmi au moins l'une de la rosacée érythémato-télangiectasique, de la rosacée papulo-pustuleuse, de la rosacée phymateuse et la rosacée oculaire.

4. Composition topique pour une utilisation selon l'une quelconque des revendications 1 à 3, dans laquelle la neurotoxine botulique est un sérotype choisi parmi les sérotypes de neurotoxine botuliques A, B, C, D, E, F ou G.

5. Composition topique pour une utilisation selon la revendication 4, dans laquelle la neurotoxine botulique est de sérotype A.

6. Composition topique pour une utilisation selon la revendication 5, dans laquelle la neurotoxine botulique est dérivée de la souche Hall.

7. Composition topique pour une utilisation selon la revendication 1, dans laquelle la neurotoxine botulique fait partie d'un complexe ayant un poids moléculaire dans la plage de 450 kDa à 900 kDa.

8. Composition topique pour une utilisation selon la revendication 5, dans laquelle la neurotoxine botulique est purifiée et a un poids moléculaire de 150 kDa.

9. Composition topique pour une utilisation selon la revendication 8, dans laquelle la quantité efficace de neurotoxine botulique est de 0,1 ng à 2,0 ng de neurotoxine botulique purifiée pour 0,5 mL à 10 mL de crème ou gel.

10. Composition topique pour une utilisation selon la revendication 8, dans laquelle la quantité efficace de neurotoxine botulique est de 1 U à environ 20 000 U de toxine botulique par cm² de surface cutanée.

11. Composition topique pour une utilisation selon la revendication 10, dans laquelle la quantité efficace de neurotoxine botulique est de 1 U à environ 10 000 U de toxine botulique par cm² de surface cutanée.

12. Composition topique pour une utilisation selon la revendication 7 ou 8, dans laquelle le support peptidique chargé positivement est choisi parmi Arg-Lys-Lys-Arg-Arg-Gln-Arg-Arg-Arg-Gly-(Lys)n-Gly-Arg-Lys-Lys-Arg-Arg-Gln-Arg-Arg-Arg (SEQ ID N° : 3) ; et Arg-Arg-Arg-Gln-Arg-Arg-Lys-Lys-Arg-Gly-(Lys)n-Gly-Arg-Arg-Arg-Gln-Arg-Arg-Lys-Lys-Arg (SEQ ID N° : 4), où n est un entier de 10 à 20.

13. Composition topique pour une utilisation selon la revendication 7 ou 8, dans laquelle le support peptidique chargé positivement est Arg-Lys-Lys-Arg-Arg-Gln-Arg-Arg-Arg-Gly-(Lys)₁₅-Gly-Arg-Lys-Lys-Arg-Arg-Gln-Arg-Arg-Arg (SEQ ID N° : 5).

14. Composition topique pour une utilisation selon la revendication 12 ou 13, la composition comprenant en outre un diluant à base de poloxamère.

15. Composition topique pour une utilisation selon la revendication 14, dans lequel le diluant à base de poloxamère est présent à 10 % à 20 % en volume.

16. Composition topique pour une utilisation selon la revendication 15, dans lequel le diluant à base de poloxamère est présent à 15 % en volume.

17. Composition topique pour une utilisation selon l'une quelconque des revendications 1 à 16, dans laquelle la neurotoxine botulique dans la composition est produite par une technique recombinante.

18. Composition topique pour une utilisation selon l'une quelconque des revendications 3 à 17, dans laquelle ledit patient est atteint de rosacée, mas pas de rhinophyma.

19. Timbre cutanée comprenant la composition topique telle que définie dans l'une quelconque des revendications 12 à 18 et un timbre pour la peau.

20. Kit pour traiter une affection de la peau **caractérisée par** une hyperréactivité vasculaire, le kit comprenant une composition topique telle que définie dans l'une quelconque des revendications 12 à 18, comprenant en outre au moins un composant choisi parmi au moins un nettoyant doux pour la peau destiné à éliminer l'excès de composition de toxine botulique après traitement, une lotion de réduction de rougeur, au moins un traitement topique supplémentaire pour la rosacée, et des produits de protection solaire avec un SPF de 15 ou plus.

21. Utilisation d'une composition telle que définie dans l'une quelconque des revendications 12 à 18, dans la fabrication d'un médicament destiné à traiter un patient atteint d'une affection de la peau **caractérisée par** une hyperréactivité vasculaire cutanée.

22. Procédé non thérapeutique destiné à traiter un patient atteint d'une affection de la peau **caractérisée par** une hyperréactivité vasculaire cutanée, comprenant l'application d'une composition topique sur l'au moins une zone affectée de la peau du patient, dans lequel la composition topique est telle que définie dans l'une quelconque des revendications 12 à 18.


### SEQUENCE LISTING

<110> Revance Therapeutics, Inc.
   Ho, Connie
   Du, Alice
   Browne, Dan
<120> METHODS AND COMPOSITIONS FOR TREATING SKIN CONDITIONS ASSOCIATED WITH VASCULAR HYPER-REACTIVITY
<130> REVA-003/01WO
<150> US 61/165,701
   <151> 2009-04-01
<160> 8
<170> PatentIn version 3.5
<210> 1
   <211> 9
   <212> PRT
   <213> Artificial Sequence
<220>
   <223> reverse HIV-TAT
<400> 1
   Arg Arg Arg Gln Arg Arg Lys Lys Arg
   1               5
<210> 2
   <211> 9
   <212> PRT
   <213> Artificial Sequence
<220>
   <223> HIV-TAT
<400> 2
   Arg Lys Lys Arg Arg Gln Arg Arg Arg
   1               5
<210> 3
   <211> 21
   <212> PRT
   <213> Artificial Sequence
<220>
   <223> translocation peptide
<220>
   <221> MISC_FEATURE
   <222> (11)..(11)
   <223> Xaa may be Lys present in 5 to 20 copies
<400> 3
   Arg Lys Lys Arg Arg Gln Arg Arg Arg Gly Xaa Gly Arg Lys Lys Arg
   1               5                   10                  15
   Arg Gln Arg Arg Arg
               20
<210> 4
   <211> 21
   <212> PRT
   <213> Artificial Sequence
<220>
   <223> translocation peptide
<220>
   <221> MISC_FEATURE
   <222> (11)..(11)
   <223> Xaa may be Lys present in 5 to 20 copies
<400> 4
   Arg Arg Arg Gln Arg Arg Lys Lys Arg Gly Xaa Gly Arg Arg Arg Gln
   1               5                   10                  15
   Arg Arg Lys Lys Arg
               20
<210> 5
   <211> 35
   <212> PRT
   <213> Artificial Sequence
<220>
   <223> translocation peptide
<400> 5
   Arg Lys Lys Arg Arg Gln Arg Arg Arg Gly Lys Lys Lys Lys Lys Lys
   1               5                   10                  15
   Lys Lys Lys Lys Lys Lys Lys Lys Lys Gly Arg Lys Lys Arg Arg Gln
               20                  25                  30
   Arg Arg Arg
           35
<210> 6
   <211> 13
   <212> PRT
   <213> Artificial Sequence
<220>
   <223> HIV-TAT peptide fragment
<220>
   <221> MISC_FEATURE
   <222> (1)..(1)
   <223> Xaa may be absent or may be Gly present in 1 to 20 copies
<220>
   <221> MISC_FEATURE
   <222> (13)..(13)
   <223> Xaa may be absent or may be Gly present in 1 to 20 copies
<400> 6
   Xaa Arg Gly Arg Asp Asp Arg Arg Gln Arg Arg Arg Xaa
   1               5                   10
<210> 7
   <211> 13
   <212> PRT
   <213> Artificial Sequence
<220>
   <223> HIV-TAT peptide fragment
<220>
   <221> MISC_FEATURE
   <222> (1)..(1)
   <223> Xaa may be absent or may be Gly present in 1 to 20 copies
<220>
   <221> MISC_FEATURE
   <222> (13)..(13)
   <223> Xaa may be absent or may be Gly present in 1 to 20 copies
<400> 7
   Xaa Tyr Gly Arg Lys Lys Arg Arg Gln Arg Arg Arg Xaa
   1               5                   10
<210> 8
   <211> 11
   <212> PRT
   <213> Artificial Sequence
<220>
   <223> HIV-TAT peptide fragment
<220>
   <221> MISC_FEATURE
   <222> (1)..(1)
   <223> Xaa may be absent or may be Gly present in 1 to 20 copies
<220>
   <221> MISC_FEATURE
   <222> (11)..(11)
   <223> Xaa may be absent or may be Gly present in 1 to 20 copies
<400> 8
   Xaa Arg Lys Lys Arg Arg Gln Arg Arg Arg Xaa
   1               5                   10